# EUROPEAN PATENT APPLICATION

(11) **EP 3 375 482 A1**
(43) Date of publication of application: **19.09.2018**
(21) Application number: 17160942.3
(22) Date of filing: 14.03.2017
(51) Int. Cl.: A61N 1/36, A61M 5/00, A61N 1/05, A61M 5/14, A61M 37/00, A61K 9/08

(54) **ACTIVE IMPLANTABLE MULTIFUNCTIONAL DEVICE ON A BIODEGRADABLE/BIORESORBABLE SCAFFOLD AND ITS MANUFACTURING PROCESSES**

(71) Applicant: Scriba Nanotecnologie S.r.l., 40128 Bologna (IT); Consiglio Nazionale Delle Ricerche, 00185 Roma (IT); Cambridge Innovation Technologies Consulting Limited, Cambridge, Cambridgeshire CB4 0WS (GB); Alma Mater Studiorum - Università di Bologna, 40126 Bologna (IT)
(72) Inventor: BISCARINI, Fabio, 40128 Bologna (IT); FOSCHI, Giulia, 40128 Bologna (IT); GRECO, Pierpaolo, 40128 Bologna (IT); MURGIA, Mauro, 00185 Roma (IT); CRAMER, Tobias, 00185 Roma (IT); GIUSTO, Elena, Cambridge, Cambridgeshire CB4 0WS (GB); DONEGA', Matteo, Cambridge, Cambridgeshire CB4 0WS (GB); PLUCHINO, Stefano, Cambridge, Cambridgeshire CB4 0WS (GB); CAMPANA, Alessandra, 40128 Bologna (IT)
(74) Representative: Villa, Livia

(57) **Abstract**

An active multifunctional implantable device (AMID) is disclosed that has proved to be able to detect, monitor and stimulate bioelectric signals in a patient by means of current/voltage pulses coupled with local delivery of fluids containing bioactive fluids. The implantable portion of the AMID is essentially made of biodegradable and bioresorbable materials, so that it is largely resorbed by the patient organism.

## Description

### FIELD OF THE INVENTION

The present invention concerns an active multifunctional implantable device (AMID) that has proved to be able to detect, monitor and stimulate bioelectric signals in a patient by means of current/voltage pulses coupled with local delivery of fluids containing bioactive fluids. The implantable portion of the AMID is essentially made of biodegradable and bioresorbable materials, so that it is largely resorbed by the patient organism.

### STATE OF THE ART

Electrical impulses accompany many of our body daily activities ranging from thought, muscular activity to vegetative processes like digestion. Abnormalities in these bioelectric signals are related to severe diseases for which modem medicine is still searching treatments. As an alternative or complement to pharmaceuticals, modem medicine has started to develop a more technological approach which is based on electrical devices (termed electroceuticals) which interface directly to muscular tissue, nerve fibers or brain circuits with the possibility of diagnosing, monitoring, and treating an array of conditions. Pathological conditions where malfunctioning of the electrical signal propagation could be addressed with electroceuticals include some neurological diseases (e.g. Parkinson's) and spinal cord injury (SCI).

In order to further develop electroceutical treatments, new generations of implantable devices have to be developed which combine the possibility to deliberately modulate bioelectric activity together with the possibility to minimize the invasiveness of the device during implantation, operation and possibly removal.

The object of the present invention is therefore to provide an implantable device that overcomes the drawbacks of the known devices and is highly effective in the treatment of neurological diseases.

### SUMMARY OF THE INVENTION

The above object has been achieved by an active multifunctional implantable device (shortly 'AMID') as claimed in claim 1.

In a further aspect, the present invention concerns manufacturing processes for making the same.

In additional aspects, the present invention concerns the use of said implantable device for the local treatment of neurological diseases.

The characteristics and the advantages of the present invention will become apparent from the following detailed description, from the working examples provided for illustrative purposes, and from the annexed drawings wherein:
- Figure 1 shows a schematic prospective and partially exfoliated view of an embodiment of the active multifunctional implantable device of the present invention,
- Figure 2 shows an exploded prospective view of the embodiment of the active multifunctional implantable device of Figure 1,
- Figure 3 shows two possible embodiments of the active multifunctional implantable device of the present invention,
- Figure 4 shows a) an exemplary screen shot of a general user interface (GUI) during a current stimulation protocol of Example 2; b) and c) exemplary details of the waveform stimuli expressed in terms of the current density induced by potential steps,

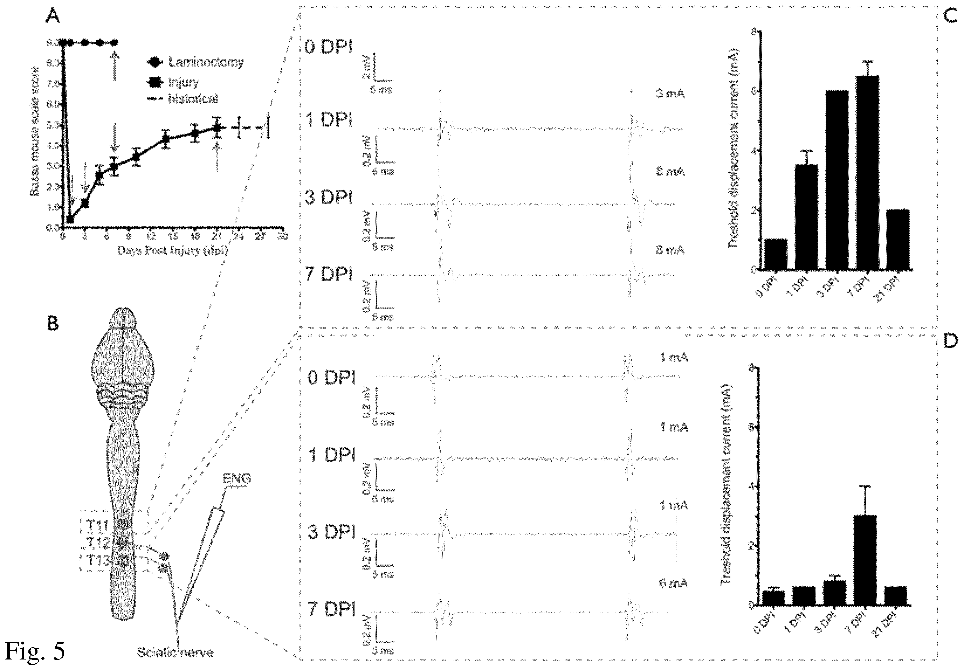

- Figure 5A shows the threshold current variation following spinal cord injury, according to the stimulation protocol and in-vivo assessment of Example 2. 10 mice have undergone surgery, 8 of them received a 70 Kdyne contusion injury and 2 received only a laminectomy (sham operated). After surgery, mice have been evaluated for their locomotor capacity (in the hind limbs) using the Basso Mouse Scale (BMS, upper left panel) until 21days post injury (DPI). At 0, 1, 3 and 7 DPI 2 mice were used for electrophysiological experiments. In particular, one mouse per time point has been anesthetized and then stimulated through the AMID above and below the injury site (T12 vertebral level), as depicted in Figure 5B. Electroneuorography (ENG) signals have been recorded from the sciatic nerve. Representative ENG traces upon stimulation of the T11 and T13 segments are shown in Figure 5C and Figure 5D respectively. The graphs on the right of Figures 5C-D show instead the threshold displacement current necessary to obtain ENG signals in the sciatic nerve,

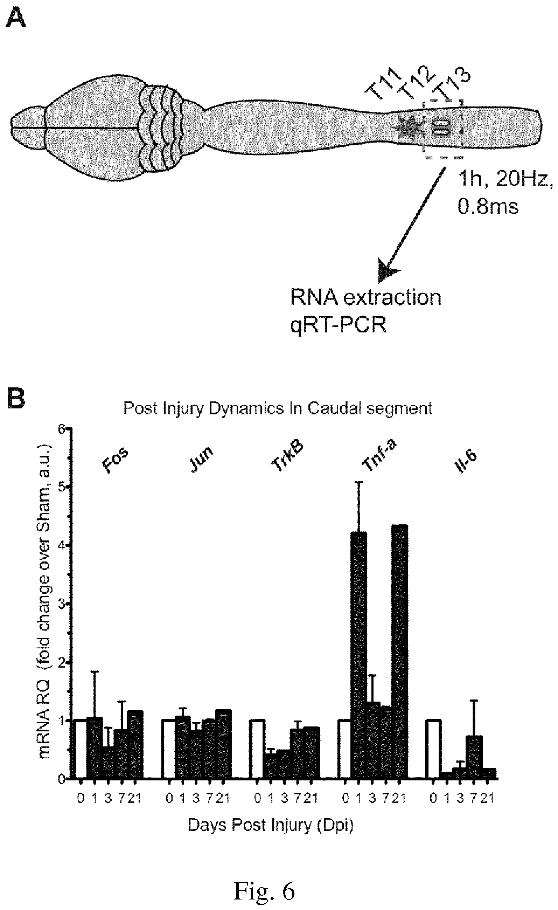

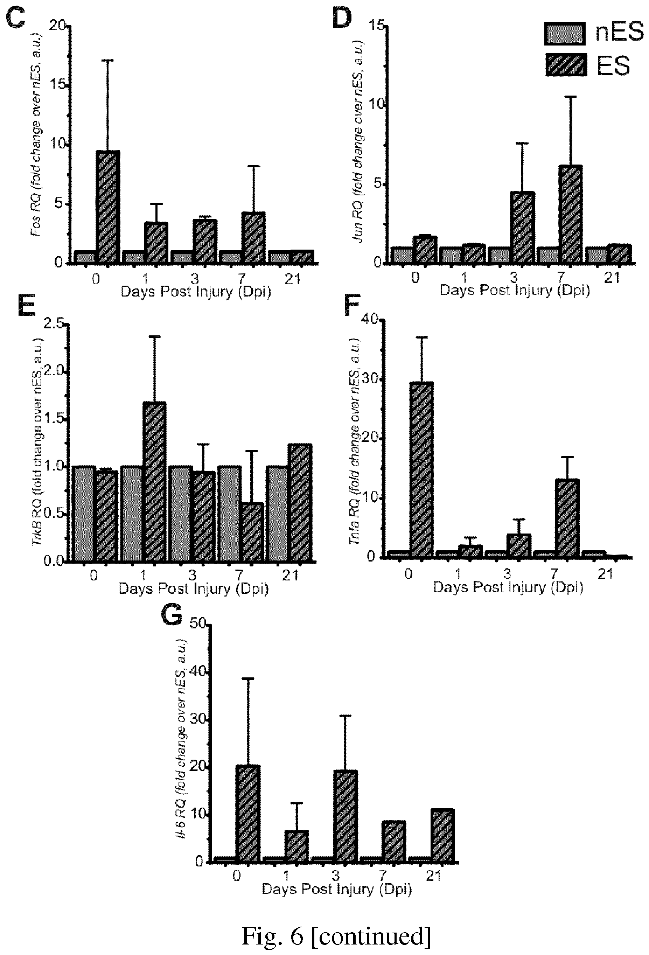

- Figure 6 shows an acute effect of electrical stimulation (ES) on the spinal segment below the injury site, according to the RNA analysis of Example 2. Twenty (20) mice were subjected to surgery, 16 of them received a 70 Kdyne contusion injury and 4 received only a laminectomy (sham operated). At 0, 1, 3, 7 and 21 DPI 2 mice were used for electrophysiological experiments. 1 of these mice was also subjected to ES paradigm (1h current stimulation) at the spinal segment below the injury site (Figure 6A). At the end of the 1h stimulation, RNA was extracted from 3 segments: T11 (Rostral), T12 (injury epicenter) and T13 (Caudal) and qRT-PCR performed. The graph of Figure 6B shows the dynamic changes of the mRNA analysed (Fos, Jun, TrkB, Tnf-a and Il-6) in the caudal segment of non-ES mice. RQ is expressed over sham-operated mice as control. The graphs of Figures 6C-G show the expression levels of the mRNA when comparing ES vs non-ES segments at different time points. Data are expressed as mean (N=2) fold change over controls.

### DETAILED DESCRIPTION OF THE INVENTION

The subject of the invention therefore is an active multifunctional implantable device (AMID) 100 having a multilayer structure comprising:
i) a biodegradable and bioresorbable protective layer 1,
ii) an active layer 2 comprising
   a biocompatible, conductive or semi-conductive, organic or inorganic compound,
   a biocompatible metal, or
   a combination thereof,
iii) a biodegradable and bioresorbable substrate layer 3, and
iv) a layer 4 provided with at least a microfluidic channel 7,
wherein said active multifunctional implantable device has an implantable portion 5 and a connecting portion 6,
wherein at least 75wt% of said implantable portion 5 is biodegradable and bioresorbable, wherein said implantable portion 5 is provided with a microfluidic outlet 8 and said connecting portion 6 is provided with a micro fluidic inlet 9 and a connector 10.

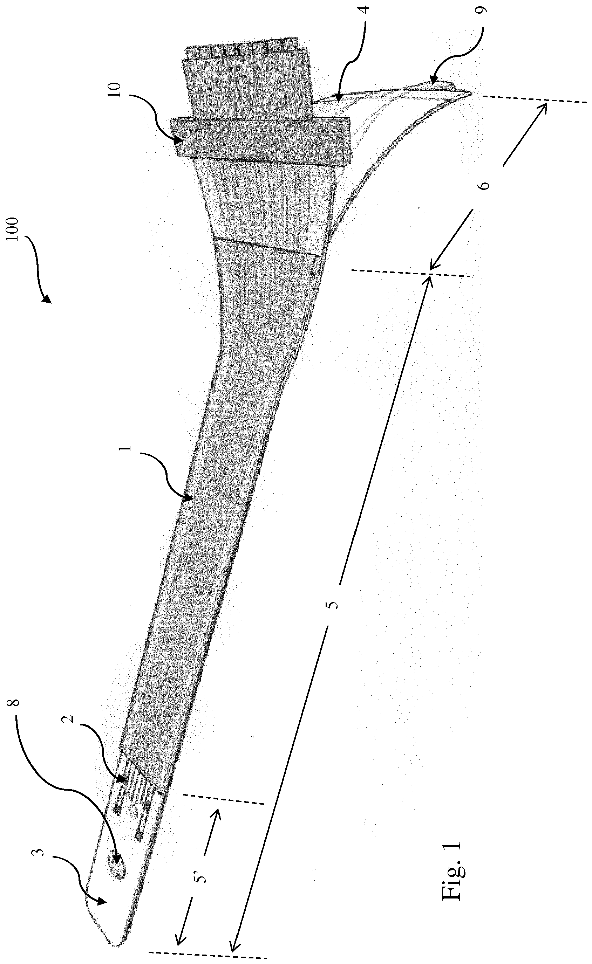

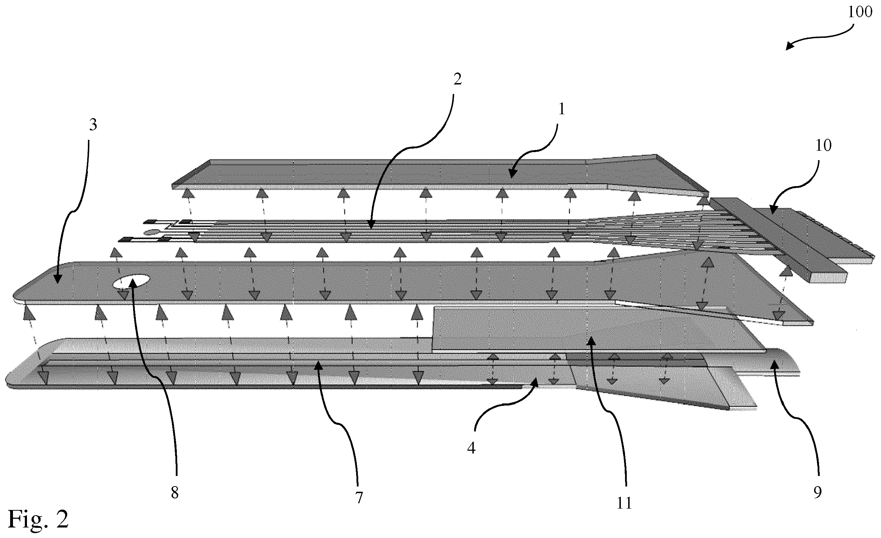

Reference is hereinafter made to the exemplary AMID shown in Figures 1 and 2.

With the term "multifunctional", it is meant that the device of the invention can deliver electric impulses to biological tissues, monitor electrical activity in biological tissues, deliver liquids, solutions or dispersions to biological tissues, extract liquids, solutions or dispersions from cavities of the animal/human body.

With the term "implantable portion", it is meant that this portion of the AMID can conform to established and/or customised surgical procedures and to reside *in vivo* without producing adverse biological reactions over extended periods of time.

The AMID of the present invention has advantageously shown to:
- exhibit long-term stability associated to high biocompatibility and safety,
- yield lower risk of a host versus graft immune response,
- mimic the local microenvironment for stem/precursor cell recruitment and differentiation,
- be able to locally monitor the functionality of the nerve cells to intervene with loco-regional therapies,
- perform local stimulation with tunable electric stimuli,
- locally deliver growth factors, neurotransmitters, and/or drugs, and especially
- have an implantable portion essentially biodegradable and bioresorbable.

With the terms "biodegradable and bioresorbable", it is meant that said implantable portion of the device does not elicit an adverse biological response once implanted *in vivo,* can be chemically degraded, e.g. hydrolytic processes, or decomposed by natural biological activity, e.g. enzymatic processes, and dissolved in or resorbed by the body without requiring any mechanical removal.

In some embodiments, at least 90wt% of said implantable portion 5 is biodegradable and bioresorbable.

In preferred embodiments, at least 95wt% of said implantable portion 5 is biodegradable and bioresorbable.

In the most preferred embodiments, at least 99wt% of said implantable portion 5 is biodegradable and bioresorbable.

It should be appreciated that said implantable portion 5 only comprises biocompatible materials. In other words, the implantable portion 5 does not comprise materials that could trigger or promote adverse events once implanted *in vivo,* such as polydimethylsiloxane, Kapton, PET, PEN, polyamide, parylene (i.e. poly(p-xylylene) polymers), chromium, aluminum, or nickel.

Suitable biodegradable and bioresorbable materials for the purposes of the present invention are polylactic acid (PLA), polyglycolic acid (PGA), polylactic-co-polyglycolic acid (PLGA), polycaprolactone (PCL), poly(hydroxyalkanoates), cellulose, chitin, chitosan, polygluconate, polylactic acid-polyethylene oxide copolymers, polyanhydride, polydioxanone, poly-phosphoester, polyhydroxybutyrate/hydroxyvalerate copolymers, poly(amino acids), poly-L-lactide, poly-D-lactide, polyglycolide, poly(alpha-hydroxy acid), their copolymers, and mixtures thereof.

Preferably, the protective layer 1, the substrate layer 3, and the layer 4 provided with at least a microfluidic channel 7 comprise at least 90wt% of said biodegradable and bioresorbable materials.

Preferably, the biodegradable and bioresorbable material is polylactic acid (PLA), polyglycolic acid (PGA), polylactic-co-polyglycolic acid (PLGA), polycaprolactone (PCL), poly(hydroxyalkanoates), cellulose, chitin, chitosan, or a mixture thereof.

In some embodiments, the protective layer 1, the substrate layer 3, and the layer 4 provided with at least a microfluidic channel 7 comprise the same biodegradable and bioresorbable materials.

In other embodiments, the protective layer 1, the substrate layer 3, and the layer 4 provided with at least a microfluidic channel 7 comprise different biodegradable and bioresorbable materials.

In preferred embodiments, the protective layer 1, the substrate layer 3, and the layer 4 provided with at least a microfluidic channel 7 comprise polylactic acid (PLA), polyglycolic acid (PGA), polylactic-co-polyglycolic acid (PLGA), polycaprolactone (PCL), poly(hydroxyalkanoates), cellulose, chitin, chitosan, or a mixture thereof.

In more preferred embodiments, the protective layer 1, the substrate layer 3, and the layer 4 provided with at least a microfluidic channel 7 comprise polylactic acid (PLA), polyglycolic acid (PGA), polylactic-co-polyglycolic acid (PLGA), polycaprolactone (PCL), or a mixture thereof.

In particularly preferred embodiments, the protective layer 1, the substrate layer 3, and the layer 4 provided with at least a microfluidic channel 7 comprise at least 90wt% of polylactic acid (PLA), polyglycolic acid (PGA), polylactic-co-polyglycolic acid (PLGA), polycaprolactone (PCL), or a mixture thereof.

The active layer 2 comprises a biocompatible, conductive or semi-conductive, organic or inorganic compound, a biocompatible metal, or a combination thereof.

In preferred embodiments, the active layer 2 comprises a combination of a biocompatible, conductive or semi-conductive, organic or inorganic compound, and a biocompatible metal.

Preferably, said biocompatible, conductive or semi-conductive, inorganic or organic compound is oligocene (including pentacene), oligothienyl (including sexithienyl), oligophenyl (including sexiphenyl), a perylene derivative (including PTDCI and PDI-8CN2), oligofluorene, PEDOT:PSS, PEDOT:tosylate, or a mixture thereof.

Preferably, said biocompatible metal is Au, Ti, Ag, Pt, Pt/Ir, or a mixture thereof.

In some embodiments, said biocompatible compound of the active layer 2 in the implantable portion 5 is functionalised for specific sensing of inflammatory biomarkers (e.g. citokines) or neurotransmitters (dopamine in the case of PD, orexine in the case of narcolepsy) using antibodies, aptamers or specifically functionalized SAMs (e.g. phenylboronic acid bound through a cysteine SAM to Au). Thus, electrodes provide local stimulation and sense physiological activity for the real time/long time monitoring of the local status. The signal measured thereby is then used to drive microfluidics' inlet of specific substances, else to operate the generator of electrical stimulus.

In some embodiments, the active layer 2 further comprises biocompatible field effect transistors whose function is to monitor bioelectric signals of the cells they are in contact with after implantation, else to measure the level of some specific molecule, such as a neurotransmitters or inflammatory biomarkers, or to supply current or potential stimuli to the interface between the transistor channel and the cells (so-called cleft). Charge accumulation and depletion of the transistor channels is controlled either by a gate electrode patterned on the substrate layer 3 (or included in the active layer 2) or by biasing both transistor electrodes with reference to the neural tissue set to ground by an external electrode. This device architecture is called organic electrochemical transistor (OECT) or electrolyte-gated organic field effect transistor (EGOFET). Capacitive coupling or ion drift upon the bias applied with respect to source-drain electrodes determine the density of charge carriers in the transistor's channel, connecting source and drain electrodes. Preferred embodiments comprise interdigitated electrodes.

In preferred embodiments, as shown in Figures 1 and 2, the implantable portion 5 comprises an end sub-portion 5', wherein the protective layer 1 is not present so that the active layer 2 including the stimulating electrodes as well as the outlet 8 of the microfluidic channel 7 can directly face and contact the targeted tissue. These embodiments enable precise and localized stimulation to be performed.

The connecting portion 6 allows the active multifunctional implantable device to be connected to an external power supply, source-measure unit (SMU) and/or potentiostat.

In preferred embodiments, the connector 10 in the connecting portion 6 is a ZIF (zero-insertion force) connector. Said connector can be coupled with front-end electronics featuring wireless communication. Said connector can be commercially available or can be made through various techniques, such as 3D printing, molding, casting by using biocompatible and/or biodegradable materials.

Electrical connection between the device and external SMU is critical. Generally, ZIF (zero insertion force) commercial connectors are used to bind the pads of the device. On the other side of the ZIF (generally used for standard electronics), small pins can be connected to flat cable, then to coaxial cables and finally, with a BNC connector, to the source measure unit (SMU).

Two AMID implantation architectures were studied.

The first AMID architecture considers the implantation of thin part of the AMID only with the ZIF connector placed outside the mouse/rat back. The advantages of this configuration reside on the very high biodegradability of the whole implanted structure, associated with the possibility of avoiding the surgery for the implant removal at the end of the treatment, and on the absence of stringent limitation on the ZIF dimension. The AMID is inserted and fixed into the ZIF. The latter is connected to a Multiplexer or directly to the SMU. Preferred connections are performed with flat cables or highly flexible architectures.

The second AMID architecture implies the implantation of the ZIF and part of the connecting architecture as well. Only the biodegradable part with the active layer is placed to interface the injury under the dura mater. On the contrary, the non-biodegradable region (ZIF and flat cable) runs more dorsal, just under the skin level. This approach is advantageous for certain applications where the device is subjected to torsional and mechanical stresses since its robustness is increased. Furthermore, the electrical connectors and microfluidics inlet can come out from the skull where a head connector is secured. Thus, the discomfort for the animal/patient can be sensitively reduced.

Additionally, in the second AMID architecture, it is important to guarantee that metallic parts from the ZIF connector or the flat cable are not exposed to the liquid, in order to prevent parasitic currents. Thus, once the electrical connection is closed, an insulating material such as medical grade silicone is preferably spread around the ZIF connector and around the first millimetres of the AMID to form a thin but tight encapsulation layer. The resulting insulating layer allows stimuli confinement. In this way, the end sub-portion 5' is the only non-encapsulated region exposed to electrophysiological environment. After a proper sterilization, the AMID is ready to be implanted.

It should be appreciated that, even if the AMID can be connected to known apparatus having their own software, a specific software running on a general user interface (GUI) has been developed for stimulation and recording operations of the transistors, as disclosed in Example 2.

In some embodiments, the multilayer structure of the active multifunctional implantable device also comprises an additional layer 11, between the substrate layer 3 and the layer 4 provided with at least a microfluidic channel 7. Suitable materials for said additional layer 11 are preferably the biocompatible and biodegradable materials listed above. Preferably, said additional layer 11 partially overlaps said at least a microfluidic channel 7, in order to define a path and to confine the injected/extracted liquids allowing for independent electric and microfluidic connectors to be realized.

The overall thickness of the multilayer structure is preferably 1-10000 µm, more preferably 1-5000 µm, even more preferably 1-1000 µm.

It should be appreciated that the materials within the AMID layers can be selected among those above described and suitably combined so as to achieve different desired degradation timings, according to the requirements set by a targeted specific pathology.

In other embodiments, also the connecting portion 6 is at least partially biodegradable and bioresorbable. Preferably, at least 50wt% of said connecting portion 6 is biodegradable and bioresorbable.

The present invention also concerns manufacturing processes for making the active multifunctional implantable device above described.

In fact, even if biodegradable and bioresorbable materials are in principle desirable, their chemical-physical characteristics negatively affect their workability and handiness in the preparation of implantable devices, so that their actual use was discouraged for long time. This was mainly because biodegradable and bioresorbable materials typically have low degradation or glass-transition temperatures, such as 50-60°C, so that low manufacturing temperatures has to be adopted. Further limitations are their solubility in non-polar or semi-polar solvents, which determine incompatibility with standard fabrication (e.g. photolithography or printing) processes, thus preventing easy manufacturing of complex architectures.

The inventors of the current invention have surprisingly found how to manufacture the active multifunctional implantable device while taking benefit of the biodegradable and bioresorbable nature of these materials, thus overcoming their chemical-physical limits.

In first embodiments, the process for manufacturing the active multifunctional implantable device comprises the steps of:
i) casting a biodegradable and bioresorbable material, thus forming a substrate layer,
ii) depositing and patterning an active layer on at least a face of said substrate layer,
iii) providing a layer with at least a microfluidic channel and sealing the same to the face of said substrate layer opposite to the face of step ii), and
iv) adding a protective layer on the face of said substrate layer of step ii).

Preferably, a biodegradable and bioresorbable layer is cast in a mould. This layer is introduced in a metal growth chamber to deposit a thin film of a metal, preferably up to a metal thickness of 20-400 nm. Different techniques for deposition can be used, such as sputtering, transfer printing, metal enhancer printing and curing, thermal evaporation and electromicro-gun induced desorption in high vacuum. Before depositing metal, a few nm (preferably 1-20 nm) Ti or Pd film can be deposited as a primer for metal adhesion on the first layer. As an alternative to the metal, either inorganic (e.g. carbon nanotubes, graphene), or organic compounds (e.g. PEDOT:PSS and poly(thienyl)s in general, poly(pyrrole)s, poly(aniline)s, poly(acetylene)s can be used.

Electrodes are then patterned by ablation of the conductive thin film by means of a stylus, or a stamp with sharp wedge-type protrusions, or preferably by means of a laser scan. The latter is effective for radiation frequencies in the IR region or in the UV region.

Alternatively, electrodes are then patterned by the evaporation of metal through a shadow mask. The mask is made of suitable materials, such as stainless steel, aluminum or plastic, and placed in close proximity to or in contact with the first layer.

Alternatively, a compatible photoresist (e.g. standard epoxy-based, hydrofluoroethers or other fluorinated based photoresists depending on the nature of the substrate) is spin coated on the surface, then patterned by photolithography to yield a negative of the desired metal pattern. After metal deposition, removal of the resist by lift-off yields patterned electrodes. In second and preferred embodiments, the process for manufacturing the active multifunctional implantable device comprises the steps of:
a) casting a water-soluble polymer layer, thus forming a sacrificial layer,
b) depositing and patterning an active layer on at least a face of said sacrificial layer,
c) casting a biodegradable and bioresorbable material on patterned conductive material, thus forming a substrate layer,
d) removing said sacrificial layer by dissolution with water,
e) providing a layer with at least a microfluidic channel and sealing the same to the face of said substrate layer opposite to the face whereon the conductive material is patterned, and
f) adding a protective layer on the face of said substrate layer whereon the conductive material is patterned.

In some embodiments, step f) is performed after step c) and step d) is performed after step e).

Preferably, the water-soluble polymer serving as sacrificial layer (SL) is deposited onto a flat (e.g.: glass, silicon wafer, quartz) support.

Preferred water-soluble polymers are poly(acrylic acid) (PAA), poly(methacrylic acid) (PMA), dextran, poly(acrylamide), poly(ethylene imine), and mixtures thereof.

The step b) of depositing and patterning an active layer can be performed as above described for step ii) of first embodiments of the process.

In additional aspects, the present invention concerns the use of above described implantable device for the local treatment of neurological diseases.

With the term "neurological disease", it is meant a pathology where an impairment of chemical and electrochemical signals causes the loss of neuronal functionality and motility control, such as spinal cord injury (SCI), Parkinson's disease (PD), narcolepsy, epilepsy, Alzheimer's disease (AD), amyotrophic lateral sclerosis, Friedreich's ataxia, Huntington's disease, Lewy body disease, spinal muscular atrophy, transverse myelitis, multiple sclerosis, chronic pain, syringomyelia, and tumors of the spinal cord.

AMID can effectively be used for the monitoring of the targeted neurodegenerative disease and the personalized loco-regional treatment of the patient. Electrodes and/or transistors at the interface with the tissue are able to monitor the neural network activity. The same electrodes and/or transistors, or additional dedicated architectures included in layer 2, can be used to perform local delivery of electrical stimuli. Frequency, duration and intensity of the stimuli can be personalized on the specific needs. Choice of delivered electrical stimulation pattern is grounded on i) clinical analysis and ii) processing of the data collected by the sensing architectures. In order to increase therapy efficacy while reducing side effect, delivery of bioactive molecules (such as anti-inflammatory, antibiotics or growth factors) is performed by means of microfluidic channels, whose outlets precisely face the targeted tissue. This allows for highly localized therapy, whose administration timings, dose and concentration are weighted on i) the clinical analysis and ii) processing of the data collected by the sensing architectures. AMID can be implanted to exploit the whole multifunctionality for the maximum impact or single functionalities (e.g. sensing, electrical stimulation delivery or drug delivery) can be exploited for specific applications. It should be understood that all aspects identified as preferred and advantageous for the implantable device are to be deemed as similarly preferred and advantageous also for the manufacturing processes and uses in treating neurological diseases of the present invention.

It should be also understood that all the combinations of preferred aspects of the implantable device, their manufacturing processes, as well as their uses in treating neurological diseases, as above reported, are to be deemed as hereby disclosed.

Below are working examples of the present invention provided for illustrative purposes.

### EXAMPLES

### Example 1.

An active multifunctional implantable device according to the present invention has been manufactured as follows.

### 1.1 Sacrificial layer (SL)

Microscopy glass slides were cleaned by sonication in acetone and isopropanol (both ultrapure grade) for 30 minutes each. A further rinse with isopropanol was used prior to be dried with N₂ stream. The glass slides were then functionalized with (3-aminopropyl)-triethoxysilane (APTES, 1 mM in ethanol) for 45 minutes. A rinse with ethanol and a N₂ stream were the final steps prior to the spin-coating of a PAA [Sigma Aldrich, 35 wt.% in H₂O, MW = 100 kDa] film. In particular, PAA, 5%(v/v) in water was spin coated with the following procedure: i) 500 rpm for 3 sec and ii) 2000 rpm for 20 sec. After a baking of 20 min at 140°C, a second layer of neutralized PAA (by means of NaOH aliquots) 3% (v/v) was spin coated with the following procedure: i) 500 rpm for 3 sec and ii) 1800 rpm for 20 sec. A final backing was performed for 20 min at 120°C. The thickness of the sacrificial layer was of about 500 nm.

### 1.2 Au deposition through a shadow mask

Mask for Au deposition, termed "shadow mask", was firstly designed with computer assisted drawing (CAD) software. Au films (50 nm thick) were thermally evaporated onto the SL in a vacuum chamber. The metal could be evaporated all over the SL surface or through a shadow mask on a defined region only, thus reducing the overall fabrication timing.

### 1.3 Au patterning by laser-scan ablation

The Au connections, leads and electrodes were achieved via laser ablation. Laser parameters, such as frequency, amplitude and power, were finely modulated in order to obtain the best compromise between high geometrical resolution and low substrate roughening. The first geometry contained 8 leads connected to 4 interdigitated couples of sources and drain electrodes along with a circular electrode placed at the centre of the active area. This electrode could be used as gate electrode of the organic transistor and/or as an independent electrode.

### 1.4 Substrate casting

A silicon frame was placed and gently pressed on the SL. It is then filled with a PLGA solution (1 mL with concentration 5% wt). The system was then heated in an oven at 55°C for 5 hours in order to complete solvent evaporation.

### 1.5 Microfluidics integration

The microfluidics was made of biodegradable and bioresorbable materials in the portion of the implantation and of biocompatible materials in the connecting portion. The outlets of the microfluidics were placed on the region where the largest concentration of bioactive molecules had to be released. The microfluidics was obtained by fixing a capillary tube in a multilayer stack comprising i) a top layer moulded on a master where a relief of the microfluidics channels had been patterned by laser scan marker or photolithography; ii) a second layer, about half the length of the top one, which was placed at the bottom of the capillary tube. The silicon capillary tube was glued with biocompatible material to the second layer. The bottom layer was sealed on the sides to the top layer, thus enclosing the capillary tube in between; iii) the substrate patterned with the electrodes. The backside of the substrate, opposite to the devices, was sealed to the top layer i).

Sealing was performed by a local pressure/temperature process by means of an ad-hoc designed machine. This guaranteed local heating around the borders of the channel. The final result was that the microfluidics inlet and ZIF electrical connector were separated apart. The microfluidic outlet was fabricated at the end of the whole process described in section 1.9.

### 1.6 Pattern release

The whole sandwich was immersed in pure water to dissolve the sacrificial layer. A complete transfer of Au pattern from PAA to PLGA was achieved in less than 120 minutes of immersion. Residual PAA on Au pattern was removed by rinsing with water. The released structure was completely dried in an oven at 35°C for 1h.

### 1.7 Lamination of a protective layer

To improve robustness and to better confine electrical stimuli, a layer was laminated on AMID. The shape and dimensions of this layer were designed and then manufactured in order to achieve both a robust coverage of the gold leads and a proper exposure of the active devices to the tissue. The lamination layer covered the whole AMID except for its two ending portions. On one side, there was a 1x1 mm² region where four interdigitated electrodes and a circular electrode were placed. On the other side, ZIF connector clamps the Au leads. It is possible to align the lamination layer with respect to the two ending sides by means of optical microscope that is then sealed on the AMID. The laminated layer did not affect the transparency of AMID. This allowed to define and restrict the effective area affected by the electrical stimulus. Consequently, current density could be tuned avoiding tissue damage. The AMID implantation requires surgical handling, therefore the presence of an additional layer makes the overall structure more robust and stable to mechanical stress.

### 1.8 PEDOT:PSS coating and patterning

The substrate was adhered to a glass slide by wetting the glass with a few droplets of water. PEDOT:PSS (as an example Clevios Jet N V2) suspension (20 µL as final volume) with added 0.2% Silquest (3-glycidoxypropyltrimethoxysilane) and 5% DMSO was spin coated on the PLGA/glass with a standard protocol (for instance: i) 500 rpm for 3 sec and ii) 2200 rpm for 20 sec on the active area (1 mm²)). The samples were then dried in an oven at 45 °C for 24 h.

PEDOT:PSS film was widely used in organic electronic devices. To optimize its performance, it was desirable to pattern PEDOT:PSS on the active area of the AMID. Different patterning techniques were available, like photolithography with dry etching, inkjet printing, transfer printing, laser assisted bioprinting, microinjection moulding in capillary and other soft lithographic techniques employing soft stamps (e.g. PDMS and agarose).

### 1.9 Shaping the AMID

The AMID was finally cut to the suited shape by means of a cutting tool, such as small scissors, a scalpel, a die-cutter, an UV laser.

In order to provide an outlet for the microfluidics in close proximity of the active devices, a needle (average diameter around 200 µm albeit this is not binding) was softly pressed on the PLGA layer.

Many steps of the described fabrication can be performed in a different order. Two examples are given: i) microfluidic sealing can occur before or after substrate release and ii) spinning and patterning of PEDOT:PSS can occur before or after application of the protective layer.

### 1.10 Electrical Connections

ZIF (zero insertion force) commercial connectors were used to bind the pads of the device. On the other side of the ZIF (generally used for standard electronics), small pins have to be somehow connected to flat cable, then to coaxial cables and finally, with a BNC connector, to the source measure unit (SMU).

Two AMID architectures were developed.

The first AMID architecture considered the implantation of thin part of the AMID only with the ZIF connector placed outside the mouse/rat back. The advantages of this configuration resided on the very high biodegradability of the whole implanted structure, associated with the possibility of avoiding the surgery for the implant removal at the end of the treatment, and on the absence of stringent limitation on the ZIF dimension. The latter is then plugged to a Multiplexer or directly to the SMU. Preferred connections are performed with flat cables or highly flexible architectures.

The second AMID architecture implied the implantation of the ZIF and part of the flat cable as well. Only the biodegradable part with the active layer was placed to interface the injury under the dura mater. On the contrary, the non-biodegradable region (ZIF and flat cable) run more dorsal, just under the skin level. This approach was advantageous for certain applications where the device is subjected to torsional and mechanical stresses since its robustness is increased. Furthermore, the electrical connectors and microfluidics inlet can come out from the skull where a head connector was secured. Thus, the discomfort for the animal/patient can be sensitively reduced.

### 1.11 Encapsulation of contacts

In the second AMID architecture, it was important to guarantee that metallic parts from the ZIF connector or the flat cable were not exposed to the liquid, so as to prevent parasitic currents. Thus, once the electrical connection was closed, medical grade silicone (140 RTV COATING, Dow Coming) was spread with a spatula around the ZIF connector and around the first millimetres of the AMID to form a thin but tight encapsulation layer. This silicone layer allowed stimuli confinement. In this way, the 1x1 mm² area, where the active devices were patterned, was the only non-insulated region exposed to electrophysiological environment. After 24 hours, upon proper sterilization, the AMID was ready to be implanted.

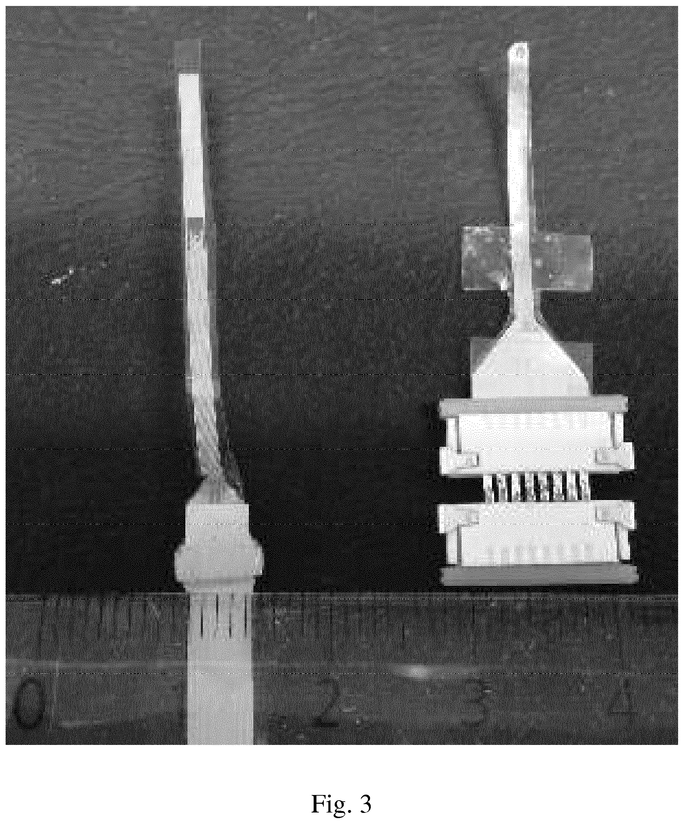

Exemplary active multifunctional implantable devices according to the present invention are shown in Figure 3.

### 1.12 Software

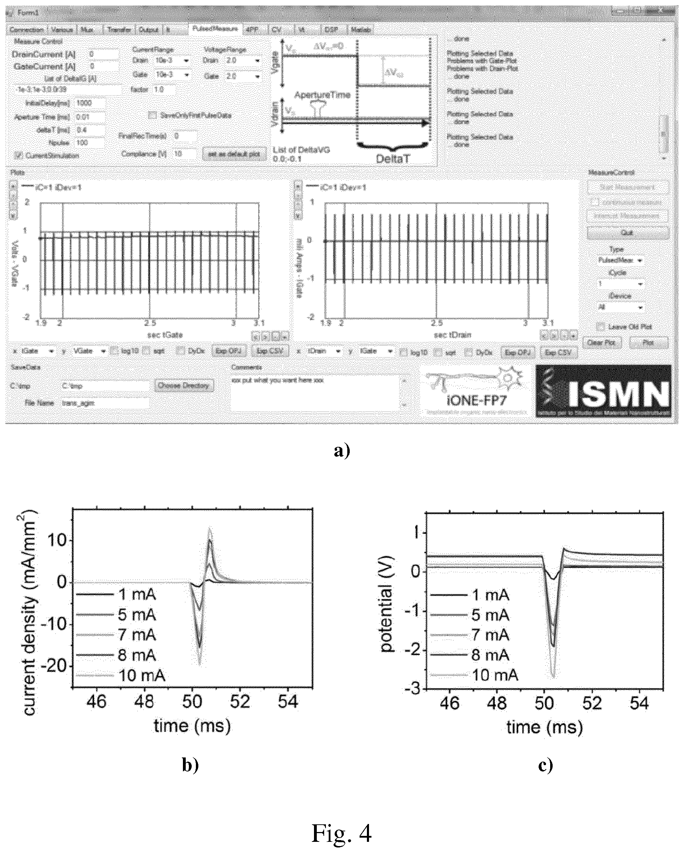

Even if the AMID can be connected to known apparatus having their own software, a specific software running on a general user interface (GUI) was developed for stimulation and recording operations of the transistors. This software environment could be easily implemented, operated by people with minimum training or automatically, and expanded into experiments of an increasing degree of complexity. The software also had a library written by the inventors to supply sequences of bio-electrically relevant stimuli and to perform sensing. An example of a stimulus sequence is shown in Figure 4. The interface allowed the most versatile choice of periodic stimuli where amplitude, duty cycle, duration, etc. can be independently set. The software could also include a library with stimulation waves.

### Example 2. Implantation protocol

All experiments involving animals were carried out in accordance with the European Communities Council Directive (86/609/EEC) and with animal welfare regulations of the UK Home Office and institutional guidelines for animal care and handling (Project license number 80/2457 to S.P.)

### 2.1 Preparation of the animal

Adult (6 to 8 weeks, 20-22 g) C57Bl/6 mice were housed in specific pathogen-free conditions in individually ventilated cages. Mice were deeply anesthetized by isoflurane inhalation (1-2%, 2 L/min). Hair on the back of the mice was shaved and swabbed with Hibiscrub4% (NVS). To prevent drying of the eyes, eye drops (Viscotears liquid gel) were applied. For the whole time of the surgery mice were placed on a heating pad to maintain the body temperature at 36-37°C during the procedure. Under a surgery microscope, a midline incision was performed over the thoracic vertebrae using a sterile scalpel. The back muscles were separated from either side of the vertebral column using an iridectomy scissor (FST Instruments, code #15000-00). Laminectomy was performed at T12 using mouse laminectomy forceps (FST Instruments, code #11223-20) with the help of a Dumont forceps (FST Instruments, code #4-11242-30) and a tissue forceps (FST Instruments, code #11021-12). Care was taken not to remove the lateral part of the vertebra at the site of laminectomy to maintain vertebral column stability. The extension of the laminectomy was approximately 1.5 mm in diameter in order to allow room for the impactor tip and it was consistent among animals. The lateral processes of T11 and T13 vertebrae were completely cleared of muscle to allow the stabilization of the vertebral column using forceps attached to the IH impactor-clamping platform.

### 2.2. Supplying a standardised spinal cord contusion injury

Contusion cord injury was then induced with a force of 70 Kdynes on the exposed cord at T12 on a total of n= 4 mice using the IH Impactor device (Precision Systems and Instrumentation, Lexington). Briefly, the mouse-impacting tip (0̸ 1.25 mm) was centred over the exposed cord in order to generate a symmetric injury. Feedback from the IH impactor, including the desired force, the actual impact force applied and the cord displacement were recorded for each mouse.

### 2.3 AMID implantation

Under general anaesthesia laboratory mice or rats are subjected to a second laminectomy, either at L1 or T10 vertebral level (additional to the T12 were the injury is applied). Laminectomy is performed by means of laminectomy forceps (FST Instruments, code #11223-20) with the help of a Dumont forceps (FST Instruments, code #4-11242-30) and a tissue forceps (FST Instruments, code #11021-12). Care was taken not to remove the lateral part of the vertebrae at the site of laminectomy to maintain vertebral column stability. Once the lamina is removed, the AMID is inserted below the L1 (or T10). To improve the stability of the implant, in some cases some drops of fibrin glue were added. The functionality of the microfluidics was tested at the moment of the implant by delivering some physiological solution. In rats, the AMID can be implanted sub-durally, by making a small incision on the dura and gently pulling the AMID in the subdural space. The device is then pulled until the active part has been positioned at the desired spinal segment. Correct positioning of the implant can be verified by applying pulses of current and monitoring paws movements. The miniaturized ZIF-connector is stabilised on the back of the spinal cord by sutures. The flat cable is then passed sub-cutaneously on the back of the laboratory mouse/rat and then externalized at the level of the head by using a custom-made head connector fixed to the skull by using mash-materials and dental cement. At the end of the procedure, the skin on the back is closed with 7-mm wound clips and animals transferred to a 27-28°C recovery incubator until complete recovery is achieved. In order to minimize animal suffering, animals receive subcutaneous injections of Buprenorphine (Temgesic, 0.1 mg/kg) as analgesic every twelve hours for the first day after the surgery. To prevent infections, animals also received subcutaneous injection of Enrofloxacin (Baytril, Bayer 5 mg/kg) for one week.

The functionality of the AMID placed in a physiological solution was assessed before starting the implantation protocol.

### 2.4. Post-surgery treatment

At the end of the procedure, the skin was closed with 7-mm wound clips and mice were transferred to a 27-28°C recovery incubator until complete recovery was achieved. To minimize animal suffering, SCI mice received subcutaneous injections of Buprenorphine (Temgesic, 0.1 mg/kg) as analgesic every twelve hours for the first two days after the surgery. To prevent infections, mice also received subcutaneous injection of Enrofloxacin (Baytril, Bayer 5 mg/kg/die) for one week. Urine was expelled by manual abdominal pressure two times a day for the first week after SCI induction and then once a week until the recovery of the bladder reflex. Mice were monitored daily and their locomotor behaviour tested twice a week according to the Basso mouse scale (BMS) score.

### 2.5 Stimulation with AMID and in-vivo assessment

Electrophysiology was performed at one-day post injury (DPI), three DPI and seven DPI. Mice with laminectomy only (without injury) were used as controls (DO). At the established time points, two mice were anaesthetized with urethane. Then the laminectomy was enlarged to allow a better exposure of the cord: both T11 and T13 were removed. An electrode was placed on the sciatic nerve and connected to an electro-neurography (ENG) recording setup.

The effects of stimulation in vivo both rostrally (T11) and caudally (T13) to the lesion site were evaluated. The AMID was placed on the spinal cord of a mouse at T11 level and stimulated with increasing currents, starting from 0.3 mA. The stimulation resulting into a clear ENG response was considered as the lowest threshold, while the stimulation causing dorsal muscles to twitch was considered as the highest threshold. Once identified the low threshold, the same stimulation caudal to the lesion (T13 vertebral level) is repeated at 20 Hz for 1 hour.

The AMID with encapsulated electrodes are stable and resistant to surgical handling. From these experiments, as expected, that a higher current level is necessary when stimulating rostral to the lesion site (compared with the caudal stimulation) (Figure 5). This is also true in healthy mice (sham controls), suggesting that part of the difference is due to the level of the stimulation, as previously described. It was also observed that the amount of current needed to generate ENG signals increases over time after injury, both for the rostral and caudal stimulation, even though there are some differences. As a matter of fact, while for the rostral stimulation there is a large difference already between 1 and 3 DPI, the conditions seem to remain much more stable for the caudal stimulation, where the differences appear at 7 DPI. However, the increase in the current threshold did not result in a great increase of ENG signal (Figure 5). The results obtained at 7DPI with the same AMID used at 1 and 3DPI were also replicated using a second AMID.

Optimum stimulation requires 2.85 times higher values of current intensity (values between 1-3.5 mA) to stimulate the cord (caudally, at T13 level) of the mouse for one hour. During this period, 40 stimulations were delivered, each composed of 0.8 ms pulses (20 Hz) for a total of 5 s. This stimulation protocol has been developed and validated in-vitro with neural progenitor stem cells (NPCs), primary neurons and macrophages.

### 2.6 RNA analysis

After the stimulation, the cord of the mouse was removed and cut into three segments (rostral, lesion epicentre and caudal), which were snap frozen for RNA analyses. The corresponding tissue was collected also from the control (non-stimulated) mouse for each time point. RNA was extracted from the tissues and analysed for the expression of mRNAs relative to Fos and Jun, which are genes known to rapidly respond to Ca²⁺ variations within the cell (therefore called "early-genes"). It is known in fact that early genes are activated quite fast via the activation of L-type voltage gated channels during neuronal activity. Electrical stimulation boosts their activity, therefore promoting increased activation of early genes. These early genes are then associated with a number of downstream activation mechanisms, among which the up-regulation of Regenerative Associated Genes (RAGs, e.g. Bdnf and TrkB). These are a family of genes that have been associated with regenerative processes within the injured nervous system. It has been shown that electrical stimulation enhances the expression of Bdnf and TrkB. Initially, as shown in Figure 6, it was analysed how the relative expression of the genes analysed changes after injury (at the different time points) when compared to non-injured tissue. No significant differences in the expression of Fos, Jun or TrkB have been observed during this time frame. On the opposite, it was possible to observe a clear increase in the expression of the mRNAs encoding for the pro-inflammatory cytokine tumour necrosis factor-α (Tnf-α) both at 1 DPI and at 21 DPI, while the pro-inflammatory cytokine interleukin-6 (Il-6) seemed to be reduced at all the time points analysed (with the exception of 7DPI, which however showed a big variation). Then it was compared the expression of the different mRNAs in electrically stimulated (ES) vs non-electrically stimulated (nES) spinal cords. The expression of Fos increases in ES mice in all the time points analysed with the exception of 21DPI, while Jun increases only at 3DPI and 7DPI. On the opposite, the expression of TrkB does not seem to increase in ES vs nES mice. As for Tnf-α, it was observed a great increase upon stimulation in the healthy mouse (DO) and a trend in its increase in injured animals over time, which, however, terminate at 21 DPI, when there is no difference between ES and nES mice. Finally, current stimulation induces an increase in the expression of interleukin-6 (Il-6) at all the time points analyzed.

The results achieved are very important to understand the appropriate parameters to use in chronic in-vivo experiments in mice with SCI. The protection of the electrodes gave extra stability to the AMID during operations: the same AMID used for at least 2-3 days of experiments still looked intact and functional. The smaller ZIF and flat cable will improve the stability of the implant in the mice.

The research performed in the background of present patent application has received funding from the European Union Seventh Framework Programme (FP7/2007-2013) under grant agreement n° 280772, project iONE-FP7, and Horizon 2020 research and innovation programme under grant agreement No 633937, project ULTRAPLACAD.

## Claims

1. An active multifunctional implantable device (100) having a multilayer structure comprising:
i) a biodegradable and bioresorbable protective layer (1),
ii) an active layer (2) comprising
a biocompatible, conductive or semi-conductive, organic or inorganic compound,
a biocompatible metal, or
a combination thereof,
iii) a biodegradable and bioresorbable substrate layer (3), and
iv) a layer (4) provided with at least a microfluidic channel (7),
wherein said active multifunctional implantable device has an implantable portion (5) and a connecting portion (6),
wherein at least 75wt% of said implantable portion (5) is biodegradable and bioresorbable, wherein said implantable portion (5) is provided with a microfluidic outlet (8) and said connecting portion (6) is provided with a microfluidic inlet (9) and a connector (10).

2. The active multifunctional implantable device (100) of claim 1, wherein at least 90wt% of said implantable portion (5) is biodegradable and bioresorbable.

3. The active multifunctional implantable device (100) of claim 2, wherein at least 95wt% of said implantable portion (5) is biodegradable and bioresorbable.

4. The active multifunctional implantable device (100) of any one of claims 1-3, wherein the protective layer (1), the substrate layer (3), and the layer (4) provided with at least a microfluidic channel (7) comprise a biodegradable and bioresorbable material, said biodegradable and bioresorbable material being polylactic acid (PLA), polyglycolic acid (PGA), polylactic-co-polyglycolic acid (PLGA), polycaprolactone (PCL), poly(hydroxyalkanoates), cellulose, chitin, chitosan, polygluconate, polylactic acid-polyethylene oxide copolymers, polyanhydride, polydioxanone, poly-phosphoester, polyhydroxybutyrate/hydroxyvalerate copolymers, poly(amino acids), poly-L-lactide, poly-D-lactide, polyglycolide, poly(alpha-hydroxy acid) or a mixture thereof.

5. The active multifunctional implantable device (100) of claim 4, wherein the protective layer (1), the substrate layer (3), and the layer (4) provided with at least a microfluidic channel (7) comprise at least 90wt% of said biodegradable and bioresorbable material.

6. The active multifunctional implantable device (100) of claim 5, wherein the protective layer (1), the substrate layer (3), and the layer (4) provided with at least a micro fluidic channel (7) comprise at least 90wt% of polylactic acid (PLA), polyglycolic acid (PGA), polylactic-co-polyglycolic acid (PLGA), polycaprolactone (PCL), or a mixture thereof.

7. The active multifunctional implantable device (100) of any one of claims 1-6, wherein, in the active layer (2), said biocompatible, conductive or semi-conductive, inorganic or organic compound is oligocene, oligothienyl, oligophenyl, a perylene derivative, oligofluorene, PEDOT:PSS, PEDOT:tosylate, or a mixture thereof, and said biocompatible metal is Au, Ti, Ag, Pt, Pt/Ir, or a mixture thereof.

8. The active multifunctional implantable device (100) of any one of claims 1-7, wherein the active layer (2) comprises a combination of a biocompatible, conductive or semi-conductive, organic or inorganic compound, and a biocompatible metal.

9. The active multifunctional implantable device (100) of any one of claims 1-8, wherein the overall thickness of the multilayer structure is 1-10000 µm, preferably 1-5000 µm, more preferably 1-1000 µm.

10. The active multifunctional implantable device (100) of any one of claims 1-9, wherein the implantable portion (5) comprises an end sub-portion (5'), wherein the protective layer (1) is not present.

11. The active multifunctional implantable device (100) of any one of claims 1-10, wherein at least 50wt% of the connecting portion 6 is biodegradable and bioresorbable.

12. A process for manufacturing the active multifunctional implantable device of any one of claims 1-11, the process comprising the steps of:
i) casting a biodegradable and bioresorbable material, thus forming a substrate layer,
ii) depositing and patterning an active layer on at least a face of said substrate layer,
iii) providing a layer with at least a micro fluidic channel and sealing the same to the face of said substrate layer opposite to the face of step ii), and
iv) adding a protective layer on the face of said substrate layer of step ii).

13. A process for manufacturing the active multifunctional implantable device of any onae of claims 1-6, the process comprising the steps of:
a) casting a water-soluble polymer layer, thus forming a sacrificial layer,
b) depositing and patterning an active layer on at least a face of said sacrificial layer,
c) casting a biodegradable and bioresorbable material on patterned conductive material, thus forming a substrate layer,
d) removing said sacrificial layer by dissolution with water,
c) providing a layer with at least a micro fluidic channel and sealing the same to the face of said substrate layer opposite to the face whereon the conductive material is patterned, and
d) adding a protective layer on the face of said substrate layer whereon the conductive material is patterned.

14. The process of claim 13, wherein the water-soluble polymer of step a) is poly(acrylic acid), poly(methacrylic acid), dextran, poly(acrylamide), poly(ethylene imine), or a mixture thereof.

15. The active multifunctional implantable device (100) of any one of claims 1-11, for use in the local treatment of neurological diseases.
